# EUROPEAN PATENT SPECIFICATION

(11) **EP 4 525 795 B1**
(45) Date of publication and mention of the grant of the patent: **03.06.2026**
(21) Application number: 23728867.5
(22) Date of filing: 09.05.2023
(51) Int. Cl.: A61F 9/007, A61M 3/02

(54) **PHACOEMULSIFICATION AND I/A SLEEVES**
PHAKOEMULSIFIKATIONS- UND I/A-HÜLSEN
PHACOÉMULSIFICATION ET MANCHONS I/A

(30) Priority: 20.05.2022 US 202263365080 P
(43) Date of publication of application: 26.03.2025
(73) Proprietor: Johnson & Johnson Surgical Vision, Inc., Irvine, CA 92618 (US)
(72) Inventor: GERG, James, Irvine, California 92618 (US)
(74) Representative: Carpmaels & Ransford LLP
(86) International application number: PCT/IB2023/054808
(87) International publication number: WO 2023/223145

(56) References cited:
- US-A- 5 725 495
- US-A- 5 755 700
- US-A1- 2006 100 653
- US-A1- 2012 116 290
- US-A1- 2015 025 451
- US-A1- 2015 038 894
- US-A1- 2015 297 344
- US-A1- 2016 192 982
- US-A1- 2020 038 008
- US-A1- 2021 060 311
- US-B1- 6 196 989
- US-B1- 8 398 578

## Description

### FIELD OF INVENTION

This invention generally relates to surgical devices used in ocular surgery and more specifically to an irrigation sleeve used with a handpiece and needle tip in ocular surgery.

### BACKGROUND

Phacoemulsification and irrigation/aspiration (I/A) handpieces facilitate removal of the natural lens during cataract surgery. During cataract surgery, a phacoemulsification handpiece and/or an IA handpiece may be coupled to an irrigation source and an aspiration pump. The handpiece may include a needle tip and an irrigation sleeve, each comprising a distal tip. The distal tip of the irrigation sleeve may comprise one or more irrigation ports, which is coupled with an irrigation source via an irrigation line, and an aspiration port at the distal tip of the needle, which is coupled with an aspiration pump via an aspiration line. Fluid from the irrigation source, which is typically an elevated bottle of balanced salt solution, is irrigated into the eye via the irrigation line and the one or more irrigation ports, and the irrigation fluid and emulsified cataractic lens material are aspirated from the eye by the aspiration pump via the aspiration port at the distal tip of the needle and the aspiration line.

Before the operation begins, the irrigation sleeve is added to the distal end of the handpiece, covering at least a portion of a needle of the phacoemulsification handpiece (thus, exposing the distal tip of the needle). A distal portion of the needle and the sleeve are then inserted through an incision in the cornea of the eye to reach the cataractic lens.

In current irrigation sleeve designs, the irrigation ports may catch on smaller incisions. As such, it would be desirable to have irrigation ports with reduced potential for snagging on incisions. It would be further desirable to have an irrigation sleeve with increased irrigation outflow to optimize chamber stability of the eye.

US 2012/0116290 A1 discloses a phacoemulsification sleeve which includes an elongated, resilient, tubular body section configured to surround a portion of a shaft of a phacoemulsification needle, the tubular body having a distal end, a proximal end, and inner surface and an outer surface. The sleeve includes at least one slitted irrigation port formed adjacent to the distal end of the body section and configured to splay in response to a drop in pressure between the inner surface and the outer surface. US 2020/038008 A1 discloses an anterior chamber maintainer (ACM) which is fastened to the cornea of a patient's eye at one or more points of contact. The ACM can be inserted through a first and a second incision. The ACM can be fastened to each of the two incisions using fasteners. The ACM includes openings to provide irrigation solution to maintain the volume of the patient's eye during cataract surgery. The shape of the openings can be customized to reduce the amount of turbulence caused by the irrigation solution. US 5 755 700 A discloses a corneal irrigation cannula for the delivery of irrigating fluid under low flow through a plurality of irrigating ports. US 2006/100653 A1 discloses an infusion sleeve for a phacoemulsification handpiece. At least three discharge ports are formed in the sleeve to provide increased flow of irrigating liquid proximate the sleeve tip. US 2015/038894 A1 discloses a phacoemulsification device including an irrigation sleeve surround an aspiration needle. Ocular material that is withdrawn from the eye is replaced with irrigation fluid that is provided through irrigation ports in the irrigation sleeve. US 5 725 495 A discloses a phacoemulsification instrument including a silicone sleeve with a built in reinforcing member and a single infusion hole.

### SUMMARY

Irrigation sleeves which maximize irrigation outflow and have a reduced potential for snagging on incisions are disclosed. More particularly, disclosed irrigation sleeves comprise webbed irrigation ports, crossed irrigation ports, axially stretched irrigation ports, and/or centering dimples. The disclosed irrigation sleeves may further provide optimal irrigation outflow for maintaining chamber stability of the eye.

In particular, the present invention provides an irrigation sleeve according to claim 1. Optional features are recited in the dependent claims. Embodiments, examples or aspects in the following disclosure which do not fall under the scope of the claims are presented for illustration purposes only and do not form part of the invention.

### BRIEF DESCRIPTION OF THE DRAWINGS

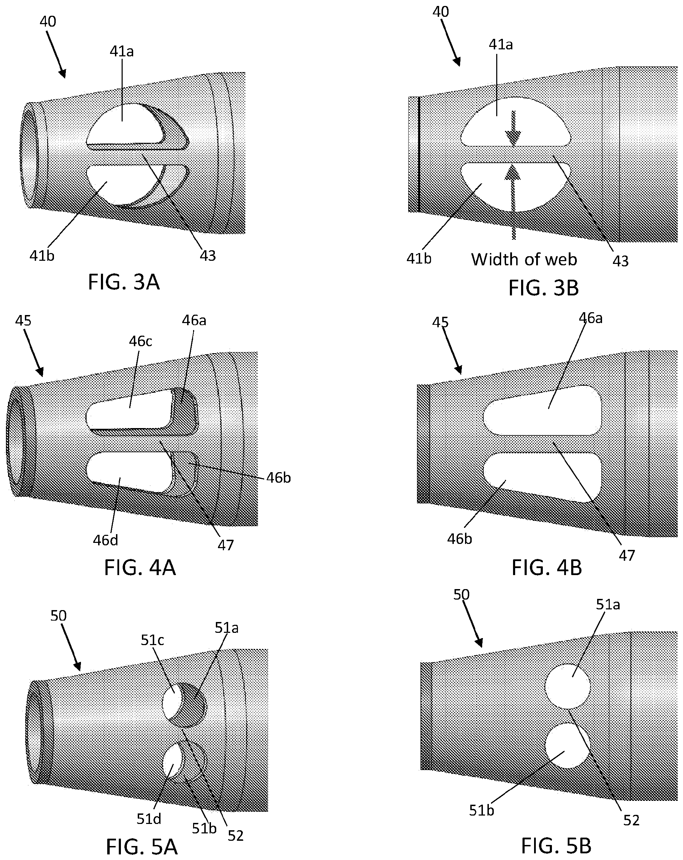

The following detailed description should be read with reference to the drawings, in which like elements in different drawings are identically numbered. The drawings, which are not necessarily to scale, depict selected embodiments and are not intended to limit the scope of the invention. Figures 3A, 3B, 4A, 4B, and 11 disclose embodiments of the invention. The remaining drawings are useful for understanding the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.
FIG. 1 is a diagram of a phacoemulsification handpiece known in the art;
FIG. 2A is a perspective view of irrigation sleeve known in the art;
FIG. 2B is a perspective view of another irrigation sleeve known in the art;
FIG. 2C is a side section view of an irrigation sleeve known in the art covering a portion of a phacoemulsification needle;
FIGs. 3A and 3B are a perspective view and a side view, respectively, of an irrigation sleeve in accordance with the present invention;
FIGs. 4A and 4B are a perspective view and a side view, respectively, of an irrigation sleeve in accordance with the present invention;
FIGs. 5A and 5B are a perspective view and a side view, respectively, of an irrigation sleeve in accordance with the present disclosure;
FIGs. 6A and 6B are a perspective view and a side view, respectively, of an irrigation sleeve in accordance with the present disclosure;
FIGs. 7A and 7B are a perspective view and a side view, respectively, of an irrigation sleeve in accordance with the present disclosure;
FIGs. 8A and 8B are a perspective view and a side view, respectively, of an irrigation sleeve in accordance with the present disclosure;
FIGs. 9A and 9B are a perspective view and a side view, respectively, of an irrigation sleeve in accordance with the present disclosure;
FIG. 10 is a side view of an irrigation sleeve in accordance with the present disclosure;
FIG. 11 is a side view of an irrigation sleeve in accordance with the present invention;
FIG. 12 is a side view of an irrigation sleeve in accordance with the present disclosure;
FIG. 13A is a perspective view of an I/A handpiece with a curved tip known in the art; and
Fig. 13B is a perspective view of an I/A handpiece with a curved tip and irrigation sleeve known in the art;
FIGs. 14A and 14B are a bottom view and a perspective view, respectively, of an irrigation sleeve in accordance with the present disclosure;
FIG. 15 is an illustration of the irrigation sleeve of FIGs. 14A and 14B encompassing a tip and within an anterior chamber of a patient's eye;
FIG. 16A is a side view of an irrigation sleeve in accordance with the present disclosure; and
FIG. 16B is a side sectional view of the irrigation sleeve of FIG. 16A.

### DETAILED DESCRIPTION OF THE PREFERRED EMBODIMENTS

Improved irrigation sleeves for phacoemulsification and irrigation/aspiration (I/A) handpieces are disclosed. All of the irrigation sleeves described below may be used with either phacoemulsification or I/A handpieces. Irrigation sleeves in accordance with the present disclosure may be made from any type of material, including, but not limited to, silicone, plastic, and/or rubber, and different sections may be made from different materials. For example, in some embodiments, the irrigations sleeve is made from silicone rubber. The irrigation sleeves may be manufactured using any manufacturing process. For example, silicone irrigation sleeves may be manufactured using silicone gum stock transfer molding and/or silicone liquid injection molding. Further, the irrigation and/or aspiration ports of irrigation sleeves in accordance with the present disclosure may be formed using various methods, including but not limited to punching, skiving, drilling, laser cutting, and/or molding.

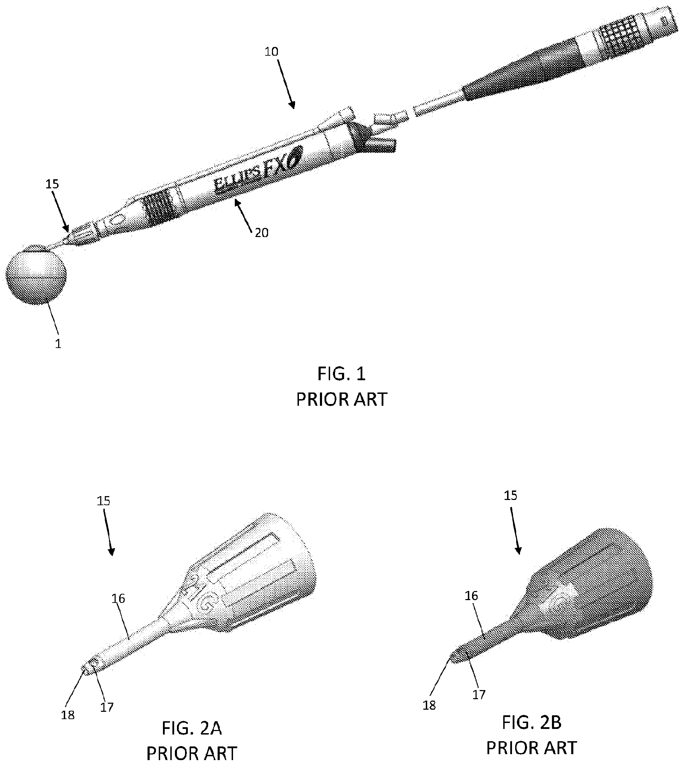

FIG. 1 is a diagram of a phacoemulsification handpiece 10 as known in the art. During ocular surgery, phacoemulsification handpiece 10 may be coupled with an irrigation source and an aspiration pump (not shown) via the proximal end of the handpiece. The handpiece 10 may include an irrigation sleeve 15.

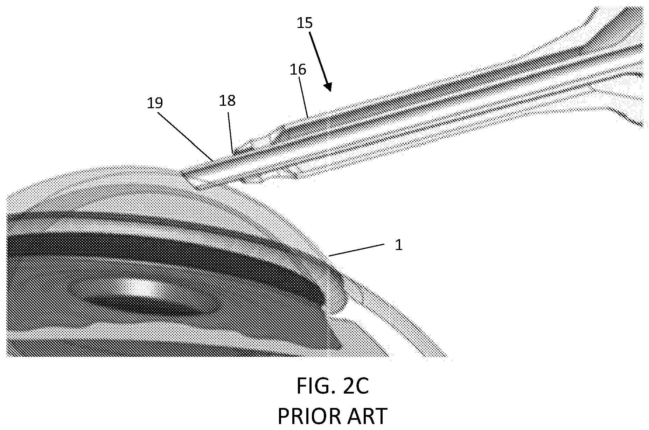

FIGs. 2A and 2B are diagrams of irrigation sleeves 15 known in the art. FIG. 2C is a diagram of an irrigation sleeve 15 known in the art covering a portion of a phacoemulsification needle 19. The irrigation sleeves 15 may comprise a distal portion 16. The distal portion 16 comprises irrigation port 17, which is coupled to an irrigation source via an irrigation line (not shown), and a distal end port or distal opening 18, which substantially surrounds the phacoemulsification needle 19, as shown in FIG. 2C. Concomitantly with the emulsification, fluid from the irrigation source, which is typically an elevated bottle of balanced salt solution, but may also be supplied via pressurized infusion or a pump, is irrigated into the eye 1 via the irrigation line and irrigation port 17, and the irrigation fluid and emulsified cataractic lens material are aspirated from the eye 1 by the aspiration pump via the phacoemulsification needle and the aspiration line.

In preparation for operation, irrigation sleeve 15 is added to the distal end of the handpiece, covering at least a portion of the needle (thus, exposing the distal tip of the needle), providing an irrigation pathway extending from the irrigation source to the irrigation port 17 via the irrigation line. The needle and a portion of the sleeve are then inserted through an incision in the cornea of the eye to reach the cataractic lens.

Improved irrigation sleeves for phacoemulsification and I/A handpieces are disclosed. Specifically, irrigation sleeves with reduced potential for catching the irrigation port on the incision and with increased irrigation outflow are disclosed. The manufacturing, materials and design of the irrigation sleeves are configured to optimize the amount of outflow while minimizing corneal snagging.

FIGs. 3A and 3B illustrate an irrigation sleeve 40 with webbed ports, according to an embodiment of the invention. The irrigation sleeve comprises a plurality of irrigation ports and a plurality of webs. In the embodiment illustrated in FIGs. 3A and 3B, irrigation sleeve 40 comprises a pair of irrigation ports 41a, 41b located opposite adjacent to each other on the same side of distal end of the irrigation sleeve 40. The irrigation ports 41a, 41b are separated by a web 43. The width of the web 43 (i.e., the space between the arrows) is less than or equal to 1.5 times the thickness of a wall of the irrigation sleeve, or less than or equal to about 0.012 inches (0.3048mm). In some embodiments, the width of web 43 (i.e., the space between the arrows) is no larger than the thickness of a wall of the irrigation sleeve. In other embodiments, the width of the web 43 is greater than or equal to the thickness of a wall of the irrigation sleeve. In some embodiments, the width of the web 43 is less than or equal to about 0.010 inches (0.254mm). In some embodiments, the width of the web 43 is less than or equal to about 0.006 inches (0.1524mm). In some embodiments, the width of the web 43 is less than or equal to 1.5 times the thickness of a wall of the irrigation sleeve or less than or equal to 0.012 inches (0.3048mm), whichever is smaller. In some embodiments, a strengthening agent and/or material may be added to one or more of the webs to increase the strength, thereby reducing the potential that the respective web will bow.

In the embodiment illustrated in FIGs. 3A and 3b, the web 43 is horizontal. The presence of the web 43 creates smaller irrigation ports, reducing the likelihood that a port will catch on an incision.

Irrigation sleeves in accordance with the present disclosure comprise irrigation ports which provide sufficient outflow. For example, in the embodiment illustrated in FIGs. 3A and 3B, the pair of irrigation ports 41a, 41b are provided. Current irrigation sleeves comprise two, relatively larger ports. By providing a greater number of smaller irrigation ports, the same or greater outflow than irrigation ports of current irrigation sleeves may be achieved. A greater outflow may be beneficial, as it may prevent the need to use higher pressures.

In the embodiment illustrated in FIGs. 3A and 3B, irrigation ports are a half-circle shape. However, the irrigation ports may be various shapes, including but not limited to rectangle, oval, trapezoid, and the like.

For example, FIGs. 4A and 4B illustrate an embodiment with irrigation sleeve 45 comprising a pair of irrigation ports 46a, 46b separated by web 47, where the pair of irrigation ports 46a, 46b are a rounded rectangular shape with a tapered side. As also illustrated in FIG. 4A, irrigation sleeve 45 may further have a second pair of irrigation ports 46c, 46d on another circumferential location on the distal end of sleeve 45.

Further, FIGs. 5A and 5B illustrate an irrigation sleeve 50 comprising a pair of irrigation ports 51a, 5b separated by web 52, where the irrigation ports are a circle shape. As also illustrated in FIG. 5A, irrigation sleeve 50 may further have a second pair of irrigation ports 51c, 51d on another circumferential location on the distal end of sleeve 50.

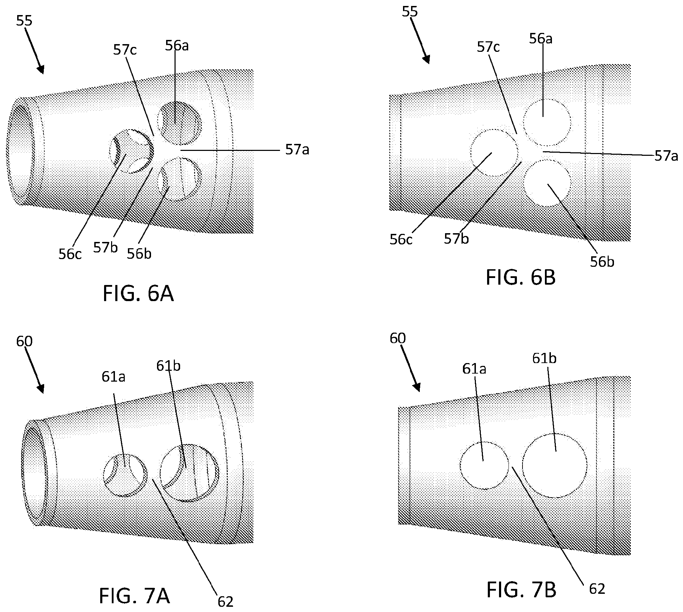

FIGs. 6A and 6B illustrate an irrigation sleeve 55 comprising two clusters of three irrigation ports located on opposite each other on the distal end of the irrigation sleeve 55. As shown in FIG. 6A, one side of irrigation sleeve 55 has three irrigation ports 56a, 56b, 56c. Although each cluster comprises three irrigation ports, more than three irrigation ports may make up a cluster. The number of irrigation ports for each cluster, the total number of irrigation ports, and/or location of the one or more ports may be determined based on, including, but not limited to, the irrigation fluid outflow desired, the size of the ports, the shape of the ports, and/or the width of the webbing.

Further, an irrigation sleeve in accordance with the present disclosure may comprise irrigation ports separated by webs that are horizontal, vertical, or diagonal, as discussed above. The sleeve illustrated in FIGs. 6A and 6B comprises a first web 57a separating irrigation ports 56a and 56b, a second web 57b separating irrigation ports 56b and 56c, and a third web 57c separating irrigation ports 56a and 56c. Similar to the embodiments discussed above, the first web 57a is horizontal. The second web 57b and third web 57c, on the other hand, are diagonal.

In the sleeve illustrated in FIGs. 7A and 7B, irrigation ports 61a, 61b are separated by a vertical web 62. Further, an irrigation sleeve in accordance with the present disclosure may comprise irrigation ports of different sizes. For example, in the sleeve illustrated in FIGs. 7A and 7B, irrigation port 61a is smaller than 61b. The different sizes may be used to achieve the desired outflow rate and direction. The different sizes may also enable easy insertion of the sleeve into the eye through an incision.

Irrigation ports in accordance with the present disclosure, such as those discussed above, may be created using punches after an irrigation sleeve has been manufactured using a technique such as injection molding. Some embodiments may be manufactured using custom punches. Some sleeves, such as the sleeve illustrated in FIGs. 5A and 5B, may be manufactured using punches that are currently used in the art. An irrigation sleeve with irrigation ports in accordance with the present disclosure, such as those discussed above, may be created using a technique such as laser cutting.

Irrigation sleeves in accordance with the present disclosure may comprise more than two irrigation ports, as discussed above. In some embodiments, the irrigation sleeve comprises at least three irrigation ports. In one embodiment where the irrigation sleeve comprises three irrigation ports, the three irrigation ports are separated by 120 degrees. In some embodiments, the at least three irrigation ports are rotationally symmetric. In other embodiments, at least one of the at least three irrigation ports is non-rotationally symmetric to at least one of the other irrigation ports. In other words, at least one of the irrigation ports may be located at a different axial position relative to the other irrigation ports to maximize wall thickness in between two ports. In further embodiments, the at least one irrigation port that is non-rotationally symmetric may comprise a different port shape, port size, and/or axial position relative to the distal tip.

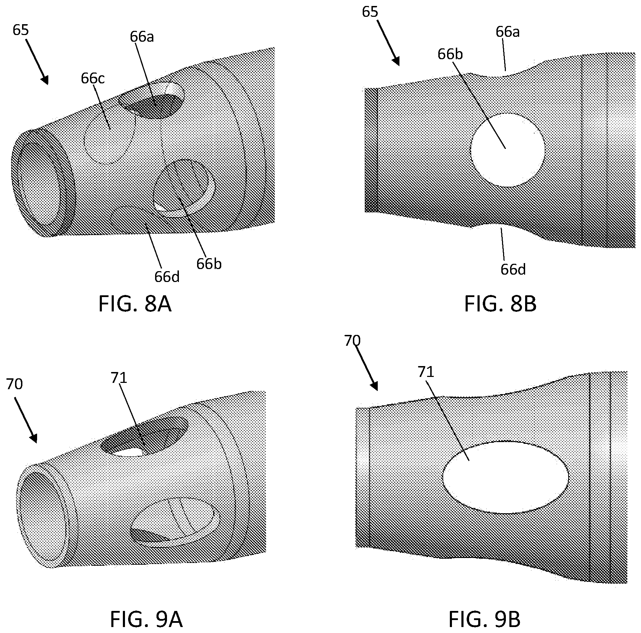

In other sleeves, the irrigation ports may comprise four crossed ports. For example, the irrigation sleeve 65 illustrated in FIGs. 8A and 8B comprises four crossed irrigation ports 66a, 66b, 66c, 66d, i.e., two pairs (66a, 66d and 66b, 66c) of irrigation ports which are directly opposite each other. The four crossed ports may be formed with only two punching steps and therefore efficient from a manufacturing standpoint. In the sleeve illustrated in FIGs. 8A and 8B, the four crossed irrigation ports 66a, 66b, 66c, 66d are separated by 90 degrees. In some sleeves, the four crossed irrigation ports 66a, 66b, 66c, 66d are separated by a distance of 0.020 inches (0.508mm).

In some embodiments, the crossed irrigation ports may have a reduced size as compared to current two port irrigation sleeves to reduce the potential for catching on corneal tissue while passing through an incision. However, the combined outflow of the four irrigation ports may be the same or greater than current irrigation sleeves with two standard-size ports. As such, irrigation sleeves in accordance with the present disclosure having more than two irrigation ports may provide sufficient outflow for maintaining chamber stability of the eye.

In the sleeve illustrated in FIGs. 8A and 8B, the irrigation ports 66 are a circle shape. However, the crossed ports may comprise various shapes, including but not limited to rectangle, circle, oval, trapezoid, and the like. For example, Figs. 9A and 9B illustrate an irrigation sleeve 70 comprising crossed irrigation ports 71 having an oval shape.

Irrigation sleeves in accordance with the present disclosure comprise axially stretched ports. The potential for corneal incision snagging may increase as a higher percentage of radial circumference is removed to form side ports. Orienting total side port open area required for sufficient irrigation flow in a mostly axial direction and minimizing side port opening in a radial direction may reduce the potential for corneal incision snagging.

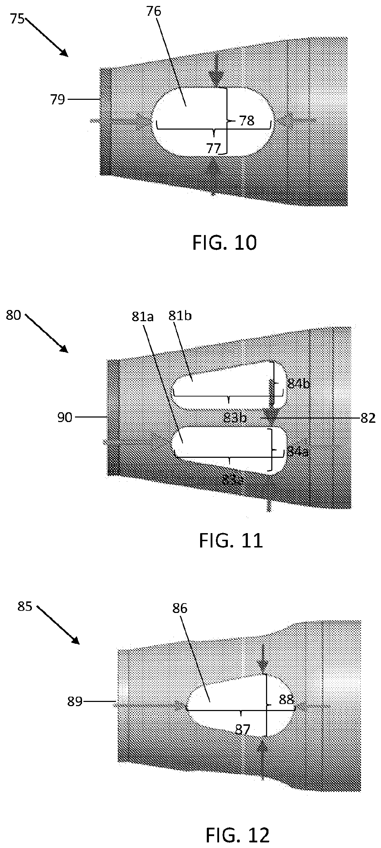

FIGs. 10-12 illustrate irrigation sleeves comprising at least one irrigation port which are elongated in an axial direction and have a minimized diametrical width. For example, the irrigation sleeve 75 illustrated in FIG. 10 comprises an axially stretched or elongated irrigation port 76. More particularly, the length of the irrigation port in the axial direction 77 is larger than the diametrical width 78 normal to the longitudinal axis.. In some embodiments, the ratio of the length of the irrigation port in the axial direction to the diametrical width of the irrigation port is about 1.5:1 or higher. In some embodiments, the ratio of the length of the irrigation port in the axial direction to the diametrical width of the irrigation port is about 3:2 or higher.

An irrigation sleeve in accordance with the present disclosure comprises two or more axially elongated ports separated by a web. For example, in the embodiment illustrated in FIG. 11, irrigation sleeve 80 comprises axially elongated irrigation ports 81a, 81b separated by web 82. More particularly, the length of each irrigation port in the axial direction 83a, 83b is larger than the diametrical width 84a, 84b of each irrigation port.

An irrigation sleeve in accordance with the present disclosure may comprise crossed irrigation ports that are axially stretched. For example, irrigation sleeve 85 of FIG. 12 comprises four crossed irrigation ports 86 which are axially stretched. More particularly, the length of irrigation port in the axial direction 87 is larger than the diametrical width 88 of the irrigation port.

An irrigation sleeve with axially elongated ports may be of various shapes, as illustrated by FIGs. 10-12. For example, the irrigation sleeve 75 of FIG. 10 comprises a distal opening 79 and irrigation ports that are a rounded rectangular shape, irrigation sleeve 80 of FIG. 11 comprises a distal opening 90 and irrigation ports that are a rounded rectangular shape with a tapered side, and irrigation sleeve 85 of FIG. 12 comprises a distal opening 89 and irrigation ports that are a teardrop shape with two tapered sides.

In some embodiments, a larger cross section of an opening is provided toward a proximal end of the irrigation sleeve. For example, in the sleeve illustrated in FIG. 12, the broader portion of the irrigation port is provided toward the proximal end of the irrigation sleeve and the narrower portion of the irrigation port is provided toward the distal end of the irrigation sleeve.

In some embodiments, one or more irrigation ports are narrower in any dimension than the distal opening, for example, distal openings 79, 90, and 89 of FIGs. 10, 11, and 12, respectively, of the irrigation sleeve. In further embodiments, one or more irrigation ports are no more than 90% of the distal opening in at least one dimension, or the smallest dimension.

In the sleeves illustrated in FIGs. 10-12, irrigation outflow of the irrigation ports is sufficient to maintain chamber stability of the eye.

In any of the embodiments discussed above, one or more irrigation ports of the irrigation sleeve or a portion of the irrigation port may be of smaller size than a needle tip used in conjunction with the irrigation sleeve to prevent the needle tip from inadvertently going through an irrigation port when irrigation sleeve is added to the distal end of the handpiece.

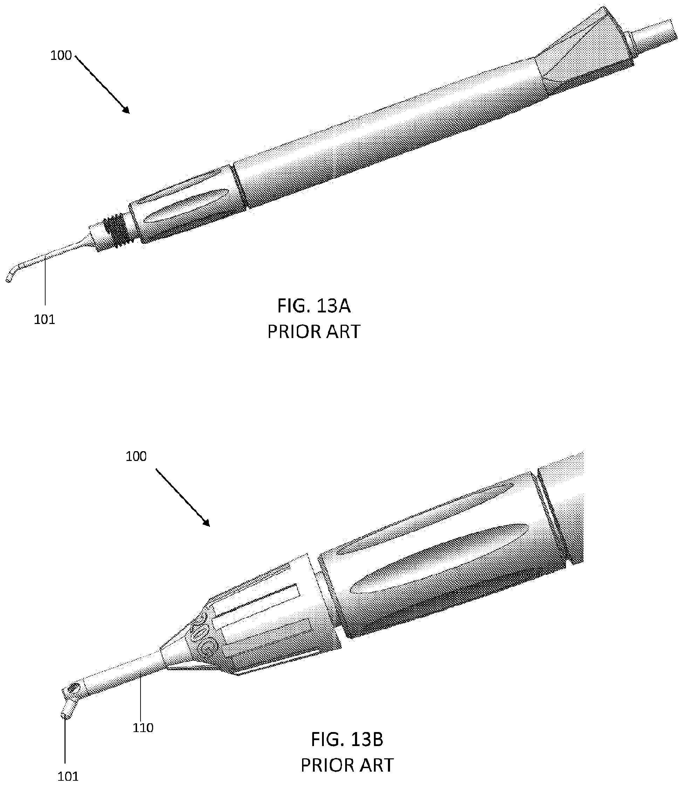

FIGs. 13A and 13B illustrate an I/A handpiece 100 with a curved needle tip 101 that is known in the art. A curved phacoemulsification tip or I/A tip may be useful in certain ocular procedures. For example, curved I/A tips may maximize access to the sub-incisional cortex. However, current irrigation sleeves, such as irrigation sleeve 110, may not symmetrically conform to a curved phacoemulsification tip or I/A tip, as illustrated in FIG. 13B. This increases the potential for (1) the distal tip of the phacoemulsification needle or I/A tip 101 to penetrate the irrigation port, (2) the outer profile of the sleeve to catch on a corneal incision, and/or (3) imbalanced and/or reduced irrigation inflow. As such, in order to address these issues an irrigation sleeve in accordance with the present disclosure may comprise two or more dimples on an inner surface of the sleeve. The dimples may maintain a centering alignment to contour both straight and curved phacoemulsification tips and I/A tips.

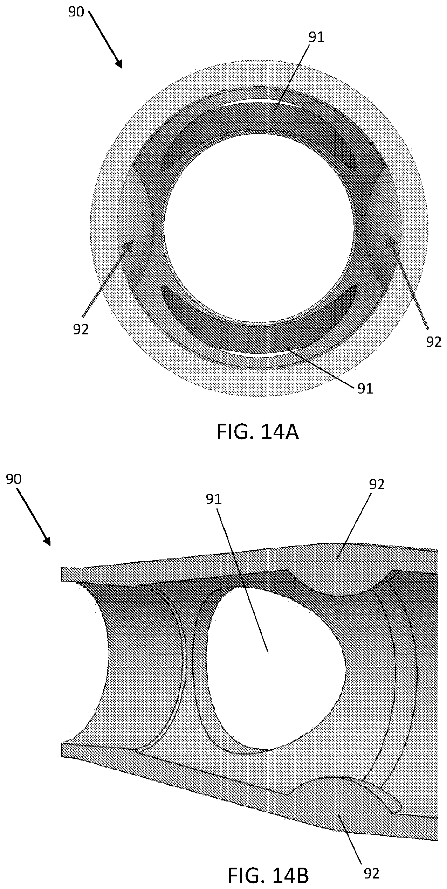

FIGs. 14A and 14B illustrate an irrigation sleeve 90 comprising two dimples 92 located on an inner surface of the sleeve. Although irrigation sleeve 90 is shown with two dimples, an irrigation sleeve in accordance with the present disclosure may comprise more than two dimples. For example, in some embodiments, the irrigation sleeve comprises three dimples located on an inner surface of the sleeve to optimize centration between the inner surface of the sleeve and an outer surface profile of the needle tip. In some embodiments, as in the sleeve illustrated in FIGs. 14A and 14B, the dimples are located between the two irrigation ports 91. In further embodiments the dimples 92 are located midway between two irrigation ports 91. For example, in the sleeve illustrated in FIGs. 14A and 14B, dimples 92 are rotationally aligned 90 degrees from the central axis of the irrigation ports 91 to guide fluid flow toward the irrigation ports 91.

In some embodiments, the irrigation sleeve may comprise three dimples in various orientations. In some embodiments, each of the three dimples are located between irrigation ports. For example, in some embodiments, two dimples may be located between two irrigation ports on a first side and one dimple may be located between the two irrigation ports on a second side opposite the first side. In some embodiments, the irrigation sleeve may comprise three dimples separated by 120 degrees.

In some embodiments, as in the sleeve illustrated in FIGs. 14A and 14B, the dimples 92 are a spherical shape. However, an irrigation sleeve in accordance with the present disclosure may comprise dimples of various shapes, such as an ovoidal shape.

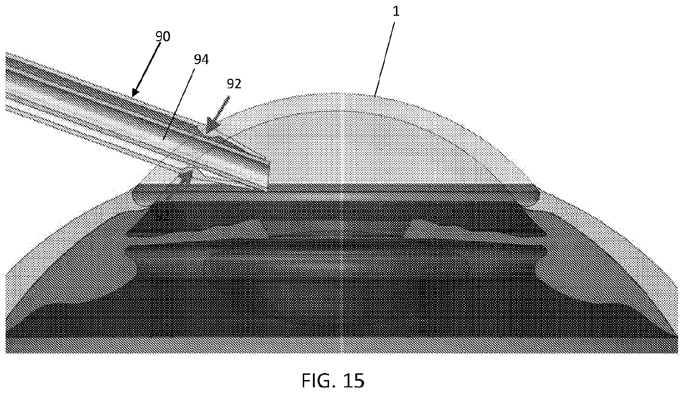

FIG. 15 illustrates irrigation sleeve 90 shown within the anterior chamber of the patient's eye 1 and encompassing a straight I/A tip 94. As shown in FIG. 15, the dimples 92 may center the irrigation sleeve 90 on the tip 94, thereby reducing the potential of the sleeve snagging on the corneal incision and ensuring uniform fluid flow. Although the irrigation sleeve 90 is shown for use with a straight I/A tip 94, irrigation sleeve 90 may be used with a curved I/A tip or a straight or curved phacoemulsification tip.

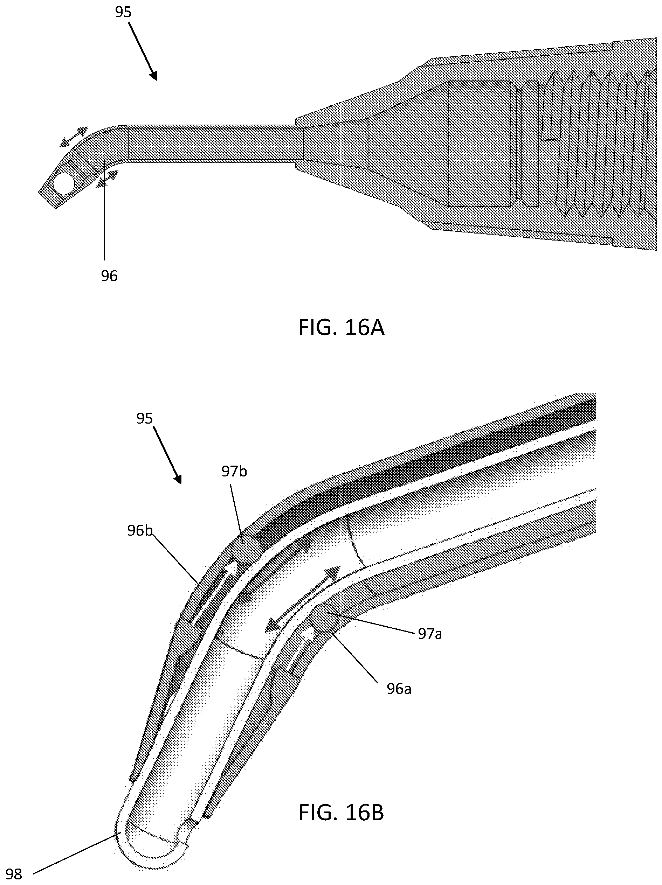

FIGs. 16A and 16B illustrate a curved irrigation sleeve 95 having a radius of curvature 96 in accordance with the present disclosure. As shown in FIG. 16B, the curved irrigation sleeve 95 may comprise curves 96a, 96b. The radius of the curves 96a, 96b may be configured to accommodate a curved phacoemulsification or I/A tip 98. The curved irrigation sleeve 95 may further comprise a plurality of dimples 97a, 97b on an inner surface of the sleeve. Although irrigation sleeve 95 is shown with two dimples 97a, 97b, an irrigation sleeve in accordance with the present disclosure may comprise more than two dimples. The dimples 97a, 97b may maintain a centering alignment to reduce the potential of the sleeve snagging on the corneal incision and ensuring uniform fluid flow. In some embodiments, as in the sleeve illustrated in FIG. 16B, the dimples 97a, 97b may be located at the midpoint of the curves 96a, 96b, respectively. In some embodiments, the dimples 97a, 97b are a spherical shape. In other embodiments, the dimples may be any number of various different shapes, such as an ovoidal shape.

In embodiments of the invention, where irrigation ports are separated by a web (e.g., embodiments illustrated in FIGs. 3A, and 4A), the web portion may be made thicker than the rest of the irrigation sleeve to increase the strength of the web, thereby reducing the potential that the web will bow, and/or create a dimple for alignment of the sleeve around the phacoemulsification and/or I/A needle tip, which may help to reduce the potential of the sleeve ports snagging on the corneal incision and provide uniform fluid flow toward the irrigation ports. In some embodiments, a strengthening agent and/or material may be added to the web. This may be done in combination with a thickening of the web in some embodiments but the use of a strengthening agent and/or materials may also be added without a thickening of the web such that a strengthened web is the same thickness as the rest of the irrigation sleeve.

It should be understood that many variations are possible based on the disclosure herein. Although features and elements are described above in particular combinations, each feature or element can be used alone without the other features and elements or in various combinations with or without other features and elements.

## Claims

1. An irrigation sleeve (40; 45: 80) for a phacoemulsification and irrigation/aspiration handpiece, the sleeve comprising:
at least one set of irrigation ports (41a, 41b; 46a, 46b; 81a, 81b) located on a distal portion of the irrigation sleeve (40; 45; 80), the at least one set of irrigation ports comprising a first port (41a; 46a; 81a) and a second port (41b; 46b; 81b), wherein the at least one set of irrigation ports are located on a single side of the irrigation sleeve (40; 45; 80) and the first port (41a; 46a; 81a) and second port (41b; 46b; 81b) are separated by a web of material (43; 47; 82), wherein
the irrigation sleeve (40; 45; 80) defines a longitudinal axis, and wherein each of the first and second ports (41a, 41b; 46a, 46b; 81a, 81b) comprises an axial length along a longitudinal axis of the irrigation sleeve and a diametrical width normal to the longitudinal axis, wherein the axial length of each of the first and second ports (41a, 41b; 46a, 46b; 81a, 81b) is larger than the diametrical width of each of the first and second ports (41a, 41b; 46a, 46b; 81a, 81b), and the web (43; 47; 82) is parallel with the longitudinal axis of the sleeve; **characterized in that**:
the first and second ports (41, 41b; 461, 46b; 81a, 81b) of the at least one set of irrigation ports have a shape selected from the group consisting of a half-circle shape, and a rounded rectangular shape with a tapered side; and **in that**
a width of the web (43; 47; 82) is less than 0.012 inches (0.3048mm) or less than 1.5 times a wall thickness of the irrigation sleeve (40; 45; 80).

2. The irrigation sleeve of claim 1, further comprising:
a second set of irrigation ports (46c, 46d) located on the distal portion of the irrigation sleeve, wherein the first set of irrigation ports (46a, 46b) and the second set of irrigation ports (46c, 46d) are located opposite each other on the irrigation sleeve (40).

3. The irrigation sleeve of any preceding claim, wherein the first set of irrigation ports (46a, 46b) and the second set of irrigation ports (46c, 46d) have a same size or have different sizes; and wherein the first set of irrigation ports (46a, 46b) and the second set of irrigation ports (46c, 46d) have a same shape or a different shape.

4. The irrigation sleeve of any preceding claim, wherein at least one of the first port (46a) and the second port (46b) has a ratio of the axial length to the diametrical width of 3:2 or more.

5. The irrigation sleeve of any preceding claim, wherein the first and second ports (46a, 46b) of the at least one set of ports have a rounded rectangular shape, wherein a distal end of each of the first and second ports (46a, 46b) is narrower than a proximal end of each of the first and second ports along the longitudinal axis of the sleeve.

6. The irrigation sleeve of any preceding claim, wherein the at least one set of irrigation ports further comprise a third port (56c), wherein the third port (56c) is separated by a second web (57b) between the first port and the third port, and wherein the third port is separated by a third web (57c) between the second port and the third port.

7. The irrigation sleeve of claim 6, wherein the second web (57b) and third web (57c) are diagonal with respect to the longitudinal axis.

8. The irrigation sleeve of any preceding claim, wherein the first port (41a; 46a; 81a) and the second port (41b; 46b; 81b) are the same size or are of different sizes.

9. The irrigation sleeve of any preceding claim, wherein the first port (41a; 46a; 81a) and the second port (41b; 46b; 81b) have a same shape or have different shapes.

## Patentansprüche

1. Spülungshülse (40; 45: 80) für ein Phakoemulsifikations- und Spülungs-/Absaughandstück, die Hülse umfassend:
mindestens einen Satz von Spülungsöffnungen (41a, 41b; 46a, 46b; 81a, 81b), die sich an einem distalen Abschnitt der Spülungshülse (40; 45; 80) befinden, der mindestens eine Satz von Spülungsöffnungen umfassend eine erste Öffnung (41a; 46a; 81a) und eine zweite Öffnung (41b; 46b; 81b), wobei sich der mindestens eine Satz von Spülungsöffnungen an einer einzigen Seite der Spülungshülse (40; 45; 80) befindet und die erste Öffnung (41a; 46a; 81a) und die zweite Öffnung (41b; 46b; 81b) durch einen Materialsteg (43; 47; 82) getrennt sind, wobei
die Spülungshülse (40; 45; 80) eine Längsachse definiert, und wobei jede der ersten und der zweiten Öffnung (41a, 41b; 46a, 46b; 81a, 81b) eine axiale Länge entlang einer Längsachse der Spülungshülse und eine diametrische Breite senkrecht zu der Längsachse umfasst, wobei die axiale Länge jeder der ersten und der zweiten Öffnung (41a, 41b; 46a, 46b; 81a, 81b) größer als die diametrische Breite jeder der ersten und der zweiten Öffnung (41a, 41b; 46a, 46b; 81a, 81b) ist und der Steg (43; 47; 82) parallel zu der Längsachse der Hülse ist;
**dadurch gekennzeichnet, dass:**
die erste und die zweite Öffnung (41, 41b; 461, 46b; 81a, 81b) des mindestens einen Satzes von Spülungsöffnungen eine Form aufweisen, die aus der Gruppe ausgewählt ist, bestehend aus einer Halbkreisform und einer abgerundeten Rechteckform mit einer sich verjüngenden Seite; und **dadurch, dass**
eine Breite des Stegs (43; 47; 82) weniger als 0,012 Zoll (0,3048 mm) oder weniger als das 1,5-Fache einer Wandstärke der Spülungshülse (40; 45; 80) beträgt.

2. Spülungshülse nach Anspruch 1, ferner umfassend:
einen zweiten Satz von Spülungsöffnungen (46c, 46d), die sich an dem distalen Abschnitt der Spülungshülse befinden, wobei sich der erste Satz von Spülungsöffnungen (46a, 46b) und der zweite Satz von Spülungsöffnungen (46c, 46d) einander gegenüberliegend an der Spülungshülse (40) befinden.

3. Spülungshülse nach einem der vorstehenden Ansprüche, wobei der erste Satz von Spülungsöffnungen (46a, 46b) und der zweite Satz von Spülungsöffnungen (46c, 46d) eine gleiche Größe aufweisen oder unterschiedliche Größen aufweisen; und wobei der erste Satz von Spülungsöffnungen (46a, 46b) und der zweite Satz von Spülungsöffnungen (46c, 46d) eine gleiche Form oder eine unterschiedliche Form aufweisen.

4. Spülungshülse nach einem der vorstehenden Ansprüche, wobei mindestens eine von der ersten Öffnung (46a) und der zweiten Öffnung (46b) ein Verhältnis der axialen Länge zu der diametralen Breite von 3 : 2 oder mehr aufweist.

5. Spülungshülse nach einem der vorstehenden Ansprüche, wobei die erste und die zweite Öffnung (46a, 46b) des mindestens einen Satzes von Öffnungen eine abgerundete Rechteckform aufweisen, wobei ein distales Ende jeder der ersten und der zweiten Öffnung (46a, 46b) schmaler als ein proximales Ende jeder der ersten und der zweiten Öffnung entlang der Längsachse der Hülse ist.

6. Spülungshülse nach einem der vorstehenden Ansprüche, wobei der mindestens eine Satz von Spülungsöffnungen ferner eine dritte Öffnung (56c) umfasst, wobei die dritte Öffnung (56c) durch einen zweiten Steg (57b) zwischen der ersten Öffnung und der dritten Öffnung getrennt ist, und wobei die dritte Öffnung durch einen dritten Steg (57c) zwischen der zweiten Öffnung und der dritten Öffnung getrennt ist.

7. Spülungshülse nach Anspruch 6, wobei der zweite Steg (57b) und der dritte Steg (57c) diagonal in Bezug auf die Längsachse sind.

8. Spülungshülse nach einem der vorstehenden Ansprüche, wobei die erste Öffnung (41a; 46a; 81a) und die zweite Öffnung (41b; 46b; 81b) gleich groß sind oder unterschiedliche Größen aufweisen.

9. Spülungshülse nach einem der vorstehenden Ansprüche, wobei die erste Öffnung (41a; 46a; 81a) und die zweite Öffnung (41b; 46b; 81b) eine gleiche Form aufweisen oder unterschiedliche Formen aufweisen.

## Revendications

1. Manchon d'irrigation (40 ; 45 ; 80) pour une pièce à main pour phacoémulsification et irrigation/aspiration, le manchon comprenant :
au moins un ensemble d'orifices d'irrigation (41a, 41b ; 46a, 46b ; 81a, 81b) situé sur une partie distale du manchon d'irrigation (40 ; 45 ; 80), l'au moins un ensemble d'orifices d'irrigation comprenant un premier orifice (41a ; 46a ; 81a) et un deuxième orifice (41b ; 46b ; 81b), dans lequel l'au moins un ensemble de orifices d'irrigation est situé sur un seul côté du manchon d'irrigation (40 ; 45 ; 80) et le premier orifice (41a ; 46a ; 81a) et le deuxième orifice (41b ; 46b ; 81b) sont séparés par une bande de matériau (43 ; 47 ; 82), dans lequel
le manchon d'irrigation (40 ; 45 ; 80) définit un axe longitudinal, et dans lequel chacun parmi les premier et deuxième orifices (41a, 41b ; 46a, 46b ; 81a, 81b) comprend une longueur axiale le long d'un axe longitudinal du manchon d'irrigation et une largeur diamétrale normale à l'axe longitudinal, dans lequel la longueur axiale de chacun parmi les premier et deuxième orifices (41a, 41b ; 46a, 46b ; 81a, 81b) est plus grande que la largeur diamétrale de chacun parmi les premier et deuxième orifices (41a, 41b ; 46a, 46b ; 81a, 81b), et la bande (43 ; 47 ; 82) est parallèle à l'axe longitudinal du manchon ; **caractérisé en ce que** :
les premier et deuxième orifices (41, 41b ; 461, 46b ; 81a, 81b) de l'au moins un ensemble d'orifices d'irrigation ont une forme choisie dans le groupe constitué d'une forme en demi-cercle et d'une forme rectangulaire arrondie avec un côté effilé ; et **en ce que**
une largeur de la bande (43 ; 47 ; 82) est inférieure à 0,012 pouce (0,3048 mm) ou inférieure à 1,5 fois une épaisseur de paroi du manchon d'irrigation (40 ; 45 ; 80).

2. Manchon d'irrigation selon la revendication 1, comprenant en outre :
un second ensemble d'orifices d'irrigation (46c, 46d) situé sur la partie distale du manchon d'irrigation, dans lequel le premier ensemble d'orifices d'irrigation (46a, 46b) et le second ensemble d'orifices d'irrigation (46c, 46d) sont situés en face l'un de l'autre sur le manchon d'irrigation (40).

3. Manchon d'irrigation selon l'une quelconque revendication précédente, dans lequel le premier ensemble d'orifices d'irrigation (46a, 46b) et le second ensemble d'orifices d'irrigation (46c, 46d) ont une même taille ou ont des tailles différentes ; et dans lequel le premier ensemble d'orifices d'irrigation (46a, 46b) et le second ensemble d'orifices d'irrigation (46c, 46d) ont une même forme ou des formes différentes.

4. Manchon d'irrigation selon l'une quelconque revendication précédente, dans lequel au moins l'un parmi le premier orifice (46a) et le deuxième orifice (46b) a un rapport entre la longueur axiale et la largeur diamétrale de 3:2 ou plus.

5. Manchon d'irrigation selon l'une quelconque revendication précédente, dans lequel les premier et deuxième orifices (46a, 46b) de l'au moins un ensemble d'orifices ont une forme rectangulaire arrondie, dans lequel une extrémité distale de chacun parmi les premier et deuxième orifices (46a, 46b) est plus étroite qu'une extrémité proximale de chacun parmi les premier et deuxième orifices le long de l'axe longitudinal du manchon.

6. Manchon d'irrigation selon l'une quelconque revendication précédente, dans lequel l'au moins un ensemble d'orifices d'irrigation comprend en outre un troisième orifice (56c), dans lequel le troisième orifice (56c) est séparé par une deuxième bande (57b) entre le premier orifice et le troisième orifice, et dans lequel le troisième orifice est séparé par une troisième bande (57c) entre le deuxième orifice et le troisième orifice.

7. Manchon d'irrigation selon la revendication 6, dans lequel la deuxième bande (57b) et la troisième bande (57c) sont diagonales par rapport à l'axe longitudinal.

8. Manchon d'irrigation selon l'une quelconque revendication précédente, dans lequel le premier orifice (41a ; 46a ; 81a) et le deuxième orifice (41b ; 46b ; 81b) sont de même taille ou sont de tailles différentes.

9. Manchon d'irrigation selon l'une quelconque revendication précédente, dans lequel le premier orifice (41a ; 46a ; 81a) et le deuxième orifice (41b ; 46b ; 81b) ont une même forme ou ont des formes différentes.
